# EUROPEAN PATENT SPECIFICATION

(11) **EP 2 958 554 B1**
(45) Date of publication and mention of the grant of the patent: **06.06.2018**
(21) Application number: 13705758.4
(22) Date of filing: 19.02.2013
(51) Int. Cl.: A61K 9/19, A61K 31/4184, A61K 47/22

(54) **STABLE COMPOSITIONS OF BENDAMUSTINE**
STABILE BENDAMUSTINZUBEREITUNG
COMPOSITION STABLE DE BENDAMUSTINE

(43) Date of publication of application: 30.12.2015
(73) Proprietor: Synthon BV, 6545 CM Nijmegen (NL)
(72) Inventor: ZALUDEK, Borek, 678 17 Blansko (CZ)
(74) Representative: Tejedor Vinent, Henar
(86) International application number: PCT/EP2013/053281
(87) International publication number: WO 2014/127802

(56) References cited:
- EP-A1- 1 982 699
- WO-A2-2006/076620
- WO-A2-2012/103226
- US-A1- 2007 116 729

## Description

Bendamustine, 4-[5-[bis(2-chloroethyl)amino]-1-methyl-2-benzimidazolyl]butyric acid of formula (I) 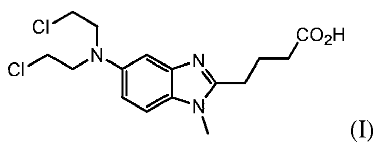 is a cytostatic agent currently approved, in the form of its hydrochloride salt, for treatment of various cancer diseases, e.g. chronic lymphocytic leukaemia. Bendamustine, including bendamustine hydrochloride, was first disclosed in DD34727. Bendamustine hydrochloride may exist, in solid state, in various polymorphic forms, which are disclosed, e.g., in WO 2009/120386. The hydrochloride product disclosed in DD 34727 is a monohydrate.

Bendamustine was developed in the early 1960s by the Institute of Microbiological and Experimental Therapy in Jena (GDR). The aim of the synthesis was to combine a purine and amino acid antagonist with an alkylating "nitrogen mustard" group (bifunctional alkylation agent). A major advantage of this compound was its water solubility.

Bendamustine is an extremely unstable compound. Due to its easy degradation in aqueous solutions, it is marketed as a lyophilized powder, i.e. a solid product, which is made by removal of a solvent (which is typically water) from a solution of bendamustine (i.e. a pre-lyophilization solution) by freeze-drying. The lyophilized product is reconstituted by adding water immediately prior to its administration by intravenous infusion to the patient. The lyophilizate is light sensitive; therefore the product is supplied in brown- or amber-coloured glass vials. In any case, lyophilized pharmaceutical compositions comprising bendamustine inherently contain various degradation products (up to several per cent in total) that are formed during manufacturing of the drug substance, during lyophilization of the aqueous solution of bendamustine and during storage.

An injectable composition of bendamustine has been disclosed in DD 80967 and DD 159289. The Jenapharm company (GDR) marketed bendamustine in the form of a sterile lyophilized powder from 1971 to 1992 under the trade name Cytostasan®. Since 1993, the lyophilized product was marketed under the names Ribomustin® and Levac®. Ribomustin® and Levac® compositions contain bendamustine hydrochloride and mannitol.

The Ribomustin® formulation comprises 25 mg or 100 mg of bendamustine hydrochloride, which is reconstituted before use by adding 10 ml or 40 ml of water for injection, respectively, i.e. resulting in a 2.5 mg/ml aqueous solution of bendamustine. WO 2006/076620 reported problems with this reconstitution, which lasts at least 15 minutes and may require as long as thirty minutes. During such lengthy reconstitution, undesired hydrolytic impurities may be formed.

The problem of formation of hydrolytic impurities, e.g. "hydroxy-bendamustine" of formula (II) 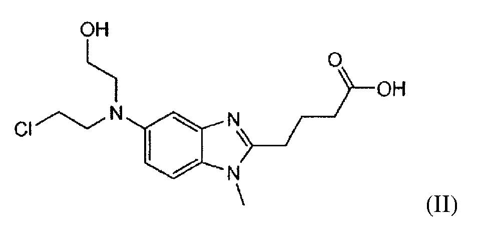 and/or "bendamustine dimer" of formula (III) 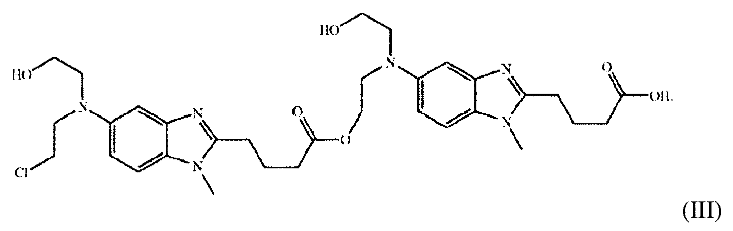 during making the bendamustine lyophilate has also been addressed in WO 2006/076620. In an attempt to solve this problem, the document suggests diluting the pre-lyophilization solution or dispersion of bendamustine in water with a stabilizing amount of at least one organic solvent selected from a fairly long list of organic solvents (see page 6, lines 17-22, and page 24, lines 4-9), wherein the content of degradation products in the lyophilized product is thereby reduced to a certain acceptable level. Preferred organic solvents include methanol, ethanol, propanol, isopropanol, butanol, and tert.butanol, more preferably tert.butanol.

Internal analysis has shown that bendamustine lyophilate sold under the brand name Treanda® by Cephalon, Inc., comprises traces of tert.butanol, therefore it may be concluded that this composition is manufactured using the stabilization process disclosed in WO 2006/076620.

It is well-known in the art that tert.butanol is the solvent of first choice among organic co-solvents that are potentially useful in the lyophilization of pharmaceuticals, so that its selection for the same purpose in WO 2006/076620 is not surprising. WO 2006/076620 discloses also a row of other solvents that may be used for stabilization of the pre-lyophilization solution (see pages 6 and 24). However, it appears that the list is rather speculative as many disclosed solvents exhibit serious disadvantages for actual industrial use. Some of them, e.g. methyl acetate or acetonitrile, have too low triple point so that lyophilization must be technically very difficult; some of them, e.g. dichloromethane or ethyl acetate, are immiscible with water so that making a monophasic solution suitable for lyophilization is hardly possible. Even most of the alcohols recommended in WO 2006/076620, particularly methanol and ethanol, exhibit a serious disadvantage as they can react with bendamustine upon formation of bendamustine esters of formula (IV) with R being methyl and ethyl, respectively.

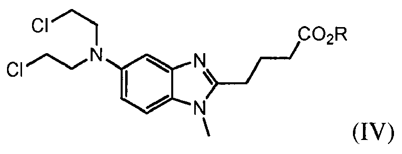

WO2012103226 describes a lyophilisation process for Bendamustine hydrochloride by lyophilising a solution containing water and/or organic solvent. Acetic acid is part of a list of possible organic solvents but the examples focus on acetone and acetonitrile as a organic solvent.

Thus, while a suggestion for stabilization of the pre-lyophilization solution for making bendamustine lyophilate exists, there is still a need for finding a robust and reliable alternative technical solution suitable for use on an industrial scale.

### BRIEF DESCRIPTION OF THE PRESENT INVENTION

The present invention relates to stabilized pharmaceutical compositions comprising bendamustine, in particular a lyophilized composition suitable for intravenous infusion.

An aspect of the present invention is a pre-lyophilization solution comprising a water soluble salt of bendamustine of formula (I), 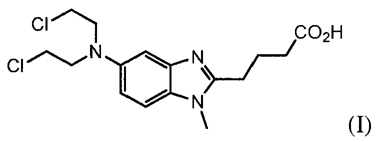 preferably bendamustine hydrochloride, and, optionally, at least one pharmaceutically acceptable excipient, dissolved in a solvent system comprising water in admixture with 20-40 volume % acetic acid.

In one embodiment, the solvent system is a binary water-dioxane mixture, preferably comprising 25-75 volume % of dioxane, more preferably 30-50 volume % of dioxane, and most preferably 35-40 volume % of dioxane.

In another embodiment, the solvent system is a ternary water-dioxane-acetic acid mixture, preferably comprising 5-40 volume % of dioxane and 5-30 volume % of acetic acid, more preferably 10-30 volume % of dioxane and 10-25 volume % of acetic acid.

In yet another embodiment, the solvent system is a binary water-acetic acid mixture, preferably comprising 5-50 volume % of acetic acid, more preferably 20-40 volume % of acetic acid.

Advantageously, the pharmaceutically acceptable excipient is a polyhydroxylated compound, preferably a sugar alcohol, most preferably mannitol.

Advantageously, the concentration of bendamustine in the pre-lyophilization solution is 3-30 mg/ml, more advantageously 10-20 mg/ml, calculated as bendamustine hydrochloride.

Advantageously, the ratio between mannitol and bendamustine hydrochloride is in the range of from 1 : 1 to 2 : 1 (w/w), preferably in the range of from 1.5 : 1 to 1.8 : 1 (w/w).

Advantageously, the pre-lyophilization solution is a sterile solution.

Another aspect of the present invention is a process for making the above-mentioned pre-lyophilization solution comprising dissolving a water soluble salt of bendamustine and, optionally, at least one pharmaceutically acceptable excipient in the above-mentioned solvent system at a temperature not exceeding ambient temperature, and filtering the solution, advantageously using a sterilization filter.

Preferably, the dissolution is performed in the absence of light and/or under an atmosphere of an inert gas.

Another aspect of the present invention is a lyophilized pharmaceutical composition comprising a water soluble salt of bendamustine and trace amounts of dioxane and/or acetic acid. Preferably, the pharmaceutical composition contains a unit dose of bendamustine hydrochloride.

Yet another aspect of the present invention is a process for making the above-mentioned lyophilized pharmaceutical composition, comprising subjecting the pre-lyophilization solution disclosed above to lyophilization.

Advantageously, said lyophilization comprises pre-cooling the solution to a temperature between -50°C and -65°C, annealing at a temperature between -28°C to -35°C, and re-cooling to a temperature between -50°C and -65°C.

### DETAILED DESCRIPTION OF THE INVENTION

The present invention relates to a stabilized pharmaceutical composition comprising a water soluble acid addition salt of bendamustine of formula (I), preferably bendamustine hydrochloride. The composition is formulated as a sterile lyophilized powder able to be reconstituted to a solution suitable for intravenous infusion, which comprises a therapeutically effective dose of bendamustine.

The stabilized composition (formulation) of the present invention is made by lyophilization (i.e. freeze-drying) of a pre-lyophilization solution comprising a water soluble salt of bendamustine dissolved in a solvent system comprising water in admixture with 20-40 volume %acetic acid. The "pre-lyophilization solution", i.e. the solution that will be subjected to lyophilization, may also comprise at least one pharmaceutically acceptable excipient. The "excipient", in accordance with common meaning, is a substance serving as an aid in making the pharmaceutical formulation without lowering or altering the therapeutic effect of the active pharmaceutical ingredient. Typically, the pre-lyophilization solution comprises a unit therapeutically effective dose of bendamustine.

Throughout the present disclosure and claims, the term "stabilized" means that the lyophilized powder, which results after lyophilization of the solution of bendamustine (hydrochloride) in the mixture of water, dioxane and/or acetic acid, contains less amount of impurities than that resulting from lyophilization of the solution of bendamustine (hydrochloride) in water alone.

Throughout the disclosure and claims, the term "dioxane" is the abbreviation for 1,4-dioxane of formula (V).

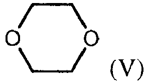

Bendamustine of formula (I) above is a known compound, which is commercially available or may be produced by methods and using equipment that is well-known in the art. Bendamustine hydrochloride is a known water soluble salt of bendamustine and is particularly useful in making compositions in accordance with the present invention. Accordingly, the compositions and processes of the invention will be illustrated on, but not limited to, bendamustine hydrochloride.

As disclosed above, it is known from WO 2006/076620 that the pre-lyophilization solution of bendamustine may be stabilized by adding certain organic solvent to the aqueous solution of bendamustine hydrochloride. This document also lists necessary properties, which such co-solvent should have for being regarded as useful. Among these properties, sufficient solubility of bendamustine in such solvent is required.

Bendamustine hydrochloride is not soluble in dioxane and presumably therefore dioxane was not listed among the useful solvents disclosed in WO 2006/076620.

Surprisingly, the present inventor found that bendamustine hydrochloride is soluble in a mixture of water and dioxane in a certain volume ratio. It was also found that the pre-lyophilization solution made by dissolving bendamustine and, optionally, other pharmaceutically acceptable excipients, in such water-dioxane mixture may be freeze-dried under common conditions using conventional lyophilization equipment. Furthermore, it was found that dioxane exhibits at least the same stabilization properties (results) as tert.butanol, i.e. the best stabilisation solvent known so far.

It was also found that stabilization properties of dioxane may even be potentiated by adding acetic acid to the water-dioxane solvent system. Thereby, the stabilizing amount of dioxane can be lowered.

Furthermore, it was found that acetic acid may even replace dioxane. This is quite surprising, as acetic acid is not fully inert to bendamustine as it may form an acetate of formula (VI) by nucleophilic substitution of one of the chlorine groups

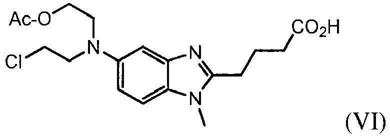

Under certain conditions, which are discussed below, such formation can however be minimized to amounts below 0.3 wt%, which is acceptable in relation to active pharmaceutical ingredient drug substance specifications.

Thus, the use of dioxane and/or acetic acid in making a lyophilized pharmaceutical composition comprising bendamustine provides a suitable alternative to tert.butanol in order to stabilize bendamustine during a process of making such a lyophilized pharmaceutical composition, as well as during storage of the pharmaceutical composition in the final dosage form. Furthermore, the use of both dioxane and acetic acid is advantageous over the use of tert.butanol in that both dioxane and acetic acid are liquids at ambient temperature, so that they can easily be handled. To the contrary, tert.butanol solidifies at temperatures below 25°C, and thus it must be handled at somewhat elevated temperature.

The pre-lyophilization solution of the present invention comprises a water soluble acid addition salt of bendamustine, preferably bendamustine hydrochloride, dissolved in a solvent system comprising water in admixture with dioxane and/or acetic acid.

In one embodiment, the solvent system is a binary water-dioxane mixture, which preferably comprises 25-75 volume % of dioxane, more preferably 30-50 volume % of dioxane, and most preferably 35-40 volume % of dioxane.

The solvent system comprising water and dioxane may also further comprise acetic acid. Thus, in another advantageous embodiment of the present invention, the solvent system is a ternary water-dioxane-acetic acid mixture. Preferably, the mixture comprises 5-40 volume % of dioxane and 5-30 volume % of acetic acid. More preferably, the mixture comprises 10-30 volume % of dioxane and 10-25 volume % of acetic acid.

In yet another embodiment of the present invention, the solvent system is a binary water-acetic acid mixture. Preferably, the mixture comprises 5-50 volume % of acetic acid, more preferably 20-40 volume % of acetic acid.

The concentration of bendamustine, calculated as bendamustine hydrochloride, in the pre-lyophilization solution composition advantageously is 3-30 mg/ml, more advantageously 10-20 mg/ml. While the stability of bendamustine in a pre-lyophilization solution comprising dioxane is roughly the same throughout the entire range mentioned above, it is recommended to use concentrations of 10 mg/ml and higher (i.e. 10-30 mg/ml) in the case when the solvent system is a water-acetic acid mixture; the stability at such concentrations is higher than that at lower concentrations.

The pre-lyophilization solution may also comprise at least one pharmaceutically acceptable excipient. Typically, the composition may comprise a water soluble bulking agent, which in certain aspects also improves physical properties of the active pharmaceutical ingredient in the final lyophilized powder. A suitable bulking agent is a polyhydroxylated compound, e.g. a carbohydrate (lactose, lactulose, sucrose, dextrose, starch etc.) or, preferably, a sugar alcohol (e.g. mannitol). The most preferred bulking agent is mannitol. The ratio between mannitol and bendamustine (hydrochloride) has a certain influence on the stability, particularly in water-acetic acid solutions. In a typical embodiment, mannitol is used in a weight amount at least equivalent to the weight amount of the bendamustine salt. Preferably, it is used in the weight amount at least equivalent to 1.5 times the weight amount of the bendamustine salt. Thus, typical ratios between mannitol and bendamustine hydrochloride are in the range of from 1 : 1 to 2 : 1 (w/w), preferably in the range of from 1.5 : 1 to 1.8 : 1 (w/w).

Other pharmaceutically acceptable excipients useful in the composition in accordance with the present invention include pH-adjustors, antioxidants, etc.

The pre-lyophilization solution of the present invention is sufficiently stable for at least 24 hours. As shown by experiments, less than 0.5 wt% of bendamustine is decomposed when storing a solution of bendamustine hydrochloride in various binary water-dioxane mixtures comprising from 25-75 volume % of dioxane at 5°C. Similar results were obtained with various ternary water-dioxane-acetic acid mixtures comprising 10-30 volume % of dioxane and 10-25 volume % of acetic acid. These experiments teach that the pre-lyophilization solution will not undergo any undesired decomposition during the making of the pre-lyophilization solution and the preparation for the lyophilization, which processes take less than 24 hours even on an industrial scale. In other similar experiments, less than 2 wt% of bendamustine was decomposed when storing a solution of bendamustine hydrochloride in binary water-acetic acid mixtures comprising from 5-50 volume % of acetic acid at 5°C. Quite surprisingly, increased concentration of acetic acid led to increased stability of the bendamustine solution.

The pre-lyophilization solution is generally made by dissolving a water soluble salt of bendamustine, preferably bendamustine hydrochloride, and any pharmaceutically acceptable excipients, in a mixture of water with dioxane and/or acetic acid, under stirring, at a temperature not exceeding ambient temperature, preferably at a temperature not exceeding 10°C, more preferably at a temperature not exceeding 5°C. The solvent should preferably be thoroughly deaerated by using an inert gas, e.g. by using nitrogen or argon, and bendamustine should preferably be treated in the absence of light. Then, the solution is optionally prefiltered through a suitable filter and is aseptically filtered through at least one sterilization filter of 0.2 microns or less mesh size into an intermediate sterile container, from which it is filled into appropriately sized vials, typically dark coloured glass vials. Each vial is advantageously filled with the volume comprising the desired unit dose amount of bendamustine, which typically corresponds to a single therapeutic dose. Typically, each vial contains such volume of the pre-lyophilization solution that comprises 10-500 mg of bendamustine hydrochloride.

It is understood that a person skilled in the art can adjust technical details of each of the steps described herein.

The final pharmaceutical composition comprising bendamustine is made by subjecting the pre-lyophilization solution disclosed above, or made by the process disclosed above, to lyophilization (i.e. freeze-drying). Lyophilization of the pre-lyophilization solution can be, in general, carried out using standard equipment as used for sterile lyophilization of aqueous pharmaceutical solutions. With respect to initial freezing, the solution of bendamustine (hydrochloride) in a water-dioxane mixture typically is frozen to about -40°C, and the solution in water-dioxane-acetic acid mixture typically is frozen to about -60°C or lower temperature.

A specific freezing regimen is advantageous for solutions of bendamustine (hydrochloride) in water-acetic acid mixtures, comprising pre-cooling the solution to a temperature between -50°C and -65°C, annealing at a temperature between -28°C to -35°C, and re-cooling to a temperature between -50°C and -65°C. This regimen is advantageous because of recrystallization of a metastable frozen phase to a stable one, resulting in easier evaporation and formation of homogeneous solid cake in subsequent drying.

Temperatures, times and pressures necessary for subsequent primary and secondary drying can be easily adjusted by those skilled in the art depending on the actual scale and choice of solvents. The pre-lyophilisation solution typically is freeze-dried until the content of all volatiles is less than 5 wt%, and then the vials are closed by a stopper, sealed, and labelled.

The sterile lyophilized pharmaceutical composition disclosed herein, being in a vial container and in an amount suitable for reconstitution and administration of one or more therapeutic doses of bendamustine, forms the pharmaceutical dosage form. The container typically is of a volume sufficient to accommodate the volume of liquid used for reconstitution. The liquid for reconstitution generally is sterile water, typically in a pharmacopoeia quality such as Water for Injection Ph. Eur. Reconstituted solutions with an amount of up to 10 mg/ml of bendamustine hydrochloride give clear and colourless solutions in line with Ph. Eur. The reconstituted solution finally is diluted with infusion solutions, e.g., with an aqueous 0.9 wt% NaCl solution, and is administered to a patient by intravenous infusion in amounts and rates prescribed by a medical doctor.

The sterile lyophilized pharmaceutical composition made from the pre-lyophilization solution prepared by the above disclosed processes comprises bendamustine and trace amounts of dioxane and/or acetic acid. The actual amounts of dioxane and/or acetic acid are detectable by common analytical methods, e.g. by GC. Advantageously, residual amounts of dioxane in the lyophilate composition are about 1 weight % or less and those of acetic acid are about 2 weight % or less, based on the total weight of the lyophilizate. The residual amounts of dioxane and/or acetic acid have a stabilizing effect on the formation of impurities during prolonged storage of the dosage form, as compared to the dosage form made by lyophilization of an aqueous solution not comprising dioxane and/or acetic acid.

The lyophilized compositions and dosage forms of the present invention are useful for treating neoplastic diseases. They can be administered alone or in combination with at least one additional anti-neoplastic agent and/or radiation therapy. No specific use limitation is relevant to the compositions of the present invention in comparison with the uses of the known and approved bendamustine lyophilized compositions.

The invention will be further illustrated by way of the following examples.

### EXAMPLES

### Example 1(comparative)

### Preparation of a freeze-dried formulation of bendamustine hydrochloride with mannitol - freeze drying from water-dioxane (38 % by volume)

An aqueous solution with a dioxane concentration of 38 % (v/v) was prepared. Subsequently, mannitol and bendamustine hydrochloride monohydrate were added and dissolved to obtain a final concentration of 12.5 mg per ml of bendamustine hydrochloride and 13 mg per ml of mannitol.

The bulk solution was prepared at 5°C in the dark. Inert atmosphere of nitrogen was used during the bulk solution preparation.

The solution was filtered through a filter of 0.2 microns, filled in vials 10R per 2 ml, and freeze-dried.

One vial of the freeze-dried product contained 25 mg of bendamustine hydrochloride and 26 mg of mannitol.

A sample of the bulk solution was stored at 5°C in a fridge for 24 hours. Results are presented in Table 1.

Analytical evaluation of the freeze-dried product is presented in Table 2. The profile of impurities of the active pharmaceutical ingredient (API) is also included.

**Table 1: Chromatographic purity of bulk solution (% , internal normalization)**

| | **API** | **T=0** | **T=24 h** |
|---|---|---|---|
| Deschloroethyl bendamustine | nd | nd | nd |
| Bendamustine hydrolytic product | 0.09 | 0.12 | 0.18 |
| UN | 0.02 | nd | nd |
| Bendamustine | 99.89 | 99.84 | 99.77 |
| UN | nd | 0.01 | 0.01 |
| Bendamustine dimer | nd | 0.02 | 0.03 |
| UN | nd | 0.01 | 0.01 |

| | | | |
|---|---|---|---|
| nd= not detected UN= unknown impurity | | | |

**Table 2: Analytical evaluation of the freeze-dried product**

| **Test** | **Initial analysis** | **Results after 3 months of storage at 40°C/75 % R.H.** |
|---|---|---|
| Colour after reconstitution, Ph. Eur. | < B9 | < B9 |
| Clarity after reconstitution, Ph. Eur. | < ref. susp. I | < ref. susp. I |
| Reconstitution time | up to 5 min | up to 5 min |
| Chromatographic purity (% IN) | | |
| Deschloroethyl bendamustine | nd | nd |
| Bendamustine hydrolytic product | 0.11 | 0.12 |
| UN | nd | 0.07 |
| Bendamustine | 99.85 | 99.71 |
| Bendamustine dimer | 0.02 | 0.02 |

| | | |
|---|---|---|
| nd= not detected UN= unknown impurity IN= internal normalization | | |

### Example 2 (comparative)

### Preparation of a freeze-dried formulation of bendamustine hydrochloride with mannitol - freeze drying from pure water

Mannitol and bendamustine hydrochochloride monohydrate were subsequently dissolved in water for injection to obtain a final concentration of 10 mg per ml of bendamustine hydrochloride and 10.4 mg per ml of mannitol. Experiment was done at 5°C in the dark. Inert atmosphere of nitrogen was used during the bulk solution preparation.

The solution was filtered through a filter of 0.2 microns, filled in vials 10R per 2.5 ml, and freeze-dried.

One vial of the freeze-dried product contained 25 mg of bendamustine hydrochloride and 20.8 mg of mannitol.

A sample of the bulk solution was stored at 5°C in a fridge for 6 hours. Results are presented in Table 3.

Analytical evaluation of the freeze-dried product is presented in Table 4. The profile of impurities of the API is also included.

**Table 3: Chromatographic purity of bulk solution (%, internal normalization)**

| | **API** | **T=0** | **T=6 h** |
|---|---|---|---|
| Deschloroethyl bendamustine | nd | nd | nd |
| Bendamustine hydrolytic product | 0.42 | 1.49 | 3.01 |
| UN | 0.02 | 0.03 | 0.03 |
| UN | 0.07 | 0.07 | 0.08 |
| UN | 0.02 | nd | nd |
| Bendamustine | 98.97 | 97.89 | 96.30 |
| UN | 0.12 | 0.08 | 0.09 |
| Bendamustine dimer | 0.34 | 0.41 | 0.47 |
| UN | 0.03 | 0.02 | 0.02 |

| | | | |
|---|---|---|---|
| nd= not detected UN = unknown impurities | | | |

**Table 4: Analytical evaluation of the freeze dried product**

| **Test** | **Initial analysis** | **Results after 3 months of storage at 40°C/75 % R.H.** |
|---|---|---|
| Colour after reconstitution, Ph. Eur. | <B9 | < B9 |
| Clarity after reconstitution, Ph. Eur. | < ref. susp. I | < ref. susp. I |
| Reconstitution time | up to 5 min | up to 5 min |
| Chromatographic purity (% IN) | | |
| Deschloroethyl bendamustine | 0.01 | 0.01 |
| Bendamustine hydrolytic product | 3.59 | 3.17 |
| UN | nd | 0.16 |
| UN | 0.03 | 0.03 |
| UN | 0.08 | 0.05 |
| Bendamustine | 95.68 | 95.95 |
| UN | 0.08 | 0.08 |
| Bendamustine dimer | 0.49 | 0.41 |
| UN | 0.02 | 0.03 |

| | | |
|---|---|---|
| nd= not detected UN= unknown impurity IN= internal normalization | | |

### Example 3 (comparative)

### Preparation of a freeze-dried formulation of bendamustine hydrochloride with mannitol - freeze drying from water-tert-butanol (40 % by volume)

An aqueous solution with tert-butanol concentration of 40 % (v/v) was prepared. Subsequently, mannitol and bendamustine hydrochochloride monohydrate were added and dissolved to obtain a final concentration of 12.5 mg per ml of bendamustine hydrochloride and 13 mg per ml of mannitol. Experiment was done at 5°C in the dark. Inert atmosphere of nitrogen was used during the bulk solution preparation.

The solution was filtered through a filter of 0.2 microns, filled in vials 10R per 2 ml, and freeze-dried.

One vial of the freeze-dried product contained 25 mg of bendamustine hydrochloride and 26 mg of mannitol.

A sample of the bulk solution was stored at 5°C in a fridge for 24 hours. Results are presented in Table 5.

Analytical evaluation of the freeze-dried product is presented in Table 6. The profile of impurities of the API is also included.

**Table 5: Chromatographic purity of bulk solution (% , internal normalization)**

| | **API** | **T=0** | **T=24 h** |
|---|---|---|---|
| Deschloroethyl bendamustine | nd | nd | nd |
| Bendamustine hydrolytic product | 0.42 | 0.45 | 0.53 |
| UN | 0.02 | 0.03 | 0.03 |
| UN | 0.07 | 0.07 | 0.07 |
| UN | 0.02 | nd | nd |
| Bendamustine | 98.97 | 98.97 | 98.89 |
| UN | 0.12 | 0.10 | 0.09 |
| Bendamustine dimer | 0.34 | 0.36 | 0.36 |
| UN | 0.03 | 0.02 | 0.02 |

| | | | |
|---|---|---|---|
| nd= not detected UN= unknown impurity | | | |

**Table 6: Analytical evaluation of the freeze-dried product**

| **Test** | **Initial analysis** | **Results after 3 months of storage at 40°C/75 % R.H.** |
|---|---|---|
| Colour after reconstitution, Ph. Eur. | < B9 | < B9 |
| Clarity after reconstitution, Ph. Eur. | < ref. susp. I | < ref. susp. I |
| Reconstitution time | up to 5 min | up to 5 min |
| Chromatographic purity (% IN) | | |
| Deschloroethyl bendamustine | nd | 0.01 |
| Bendamustine hydrolytic product | 0.45 | 0.43 |
| UN | nd | 0.07 |
| UN | 0.03 | 0.03 |
| UN | 0.07 | 0.08 |
| Bendamustine | 98.96 | 98.67 |
| UN | 0.10 | 0.09 |
| Bendamustine dimer | 0.36 | 0.48 |
| UN | 0.02 | 0.02 |

| | | |
|---|---|---|
| nd= not detected UN= unknown impurity IN= internal normalization | | |

### Example 4

### Preparation of a freeze-dried formulation of bendamustine hydrochloride with mannitol - freeze drying from water-acetic acid (25 % by volume)

An aqueous solution with acetic acid 25 % (v/v) was prepared. Subsequently, mannitol and bendamustine hydrochloride monohydrate were added and dissolved to obtain a final concentration of 12.5 mg per ml of bendamustine hydrochloride and 20 mg per ml of mannitol. Experiment was done at 5°C in the dark. Inert atmosphere of nitrogen was used during the bulk solution preparation.

The solution was filtered through a filter of 0.2 microns, filled in tubular glass vials 50 ml per 8 ml, and freeze-dried (pre-cooling to -50°C, annealing to -31°C and re-cooling to -50°C).

One vial of the freeze-dried product contained 100 mg of bendamustine hydrochloride and 160 mg of mannitol.

A sample of the bulk solution was stored at 5°C in a fridge for 24 hours. Results are presented in Table 7.

Analytical evaluation of the freeze-dried product is presented in Table 8.

**Table 7: Chromatographic purity of bulk solution (%, internal normalization)**

| | **API** | **T=0** | **T=24h** |
|---|---|---|---|
| Deschloroethyl bendamustine | nd | nd | nd |
| Bendamustine hydrolytic product | 0.15 | 0.24 | 0.62 |
| UN | 0.01 | 0.01 | 0.01 |
| UN | 0.24 | 0.24 | 0.24 |
| Bendamustine acetyl deriv. | 0.02 | 0.02 | 0.13 |
| UN | 0.02 | 0.02 | 0.02 |
| Bendamustine | 99.22 | 99.13 | 98.66 |
| UN | 0.01 | 0.01 | 0.01 |
| Bendamustine dimer | 0.22 | 0.22 | 0.21 |
| UN | 0.14 | 0.13 | 0.12 |

| | | | |
|---|---|---|---|
| nd= not detected UN= unknown impurity | | | |

**Table 8: Analytical evaluation of the freeze-dried product**

| **Test** | **Initial analysis** | **Results after 3 months of storage at 40°C/75 % R.H.** |
|---|---|---|
| Colour after reconstitution, Ph. Eur. | <B9 | < B9 |
| Clarity after reconstitution, Ph. Eur. | < ref. susp. I | < ref. susp. I |
| Reconstitution time | up to 5 minutes. | up to 5 minutes. |
| Chromatographic purity (% IN) | | |
| Deschloroethyl bendamustine | nd | nd |
| Bendamustine hydrolytic product | 0.33 | 0.33 |
| UN | 0.01 | 0.01 |
| UN | 0.25 | 0.25 |
| Bendamustine acetyl deriv. | 0.05 | 0.07 |
| UN | nd | 0.06 |
| UN | 0.02 | 0.02 |
| Bendamustine | 98.97 | 98.87 |
| UN | 0.01 | 0.01 |
| Bendamustine dimer | 0.23 | 0.26 |
| UN | 0.13 | 0.09 |

| | | |
|---|---|---|
| nd= not detected UN= unknown impurity IN = internal normalization | | |

## Claims

1. A pre-lyophilization solution comprising a water soluble salt of bendamustine of formula (I), 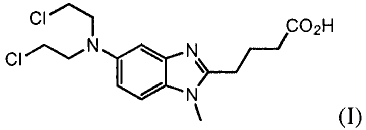 preferably bendamustine hydrochloride, dissolved in a solvent system comprising water in admixture with 20-40 volume % acetic acid.

2. The solution according to claim 1, further comprising at least one pharmaceutically acceptable excipient.

3. The solution according to claim 2, wherein the excipient is a polyhydroxylated compound, preferably a sugar alcohol, most preferably mannitol.

4. The solution according to any one of claims 1-3, wherein the concentration of bendamustine in the pre-lyophilization solution is 3-30 mg/ml, more advantageously 10-20 mg/ml, calculated as bendamustine hydrochloride.

5. The solution according to any one of claims 1-4, wherein the ratio between mannitol and bendamustine hydrochloride is in the range of from 1 : 1 to 2 : 1 (w/w), preferably in the range of from 1.5 : 1 to 1.8 : 1 (w/w).

6. The solution according to any one of claims 1-5, which is a sterile solution.

7. A process for making the pre-lyophilization solution according to any one of claims 1-6, comprising dissolving a water soluble salt of bendamustine and, optionally, at least one pharmaceutically acceptable excipient, in a solvent system comprising water in admixture with acetic acid at a temperature not exceeding ambient temperature, and filtering the solution.

8. The process according to claim 7, wherein the dissolution is performed in the absence of light and/or under an atmosphere of an inert gas.

9. A process for making a pharmaceutical composition, comprising subjecting the pre-lyophilization solution according to any one of claims 1 - 6 to lyophilization.

10. The process according to claim 9, comprising pre-cooling a solution of bendamustine hydrochloride in water-acetic acid pre-cooling the solution to a temperature between -50°C and -65°C, annealing at a temperature between -28°C to -35°C, and re-cooling to a temperature between -50°C and -65°C.

## Patentansprüche

1. Prälyophilisierungslösung, die ein wasserlösliches Salz von Bendamustin der Formel (I) umfasst, 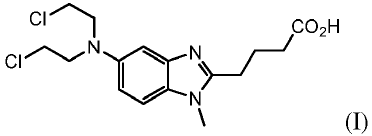 bevorzugt Bendamustinhydrochlorid, gelöst in einem Lösungsmittelsystem, das Wasser im Gemisch mit 20-40 Volumen-% Essigsäure umfasst.

2. Lösung nach Anspruch 1, die weiterhin mindestens einen pharmazeutisch verträglichen Hilfsstoff umfasst.

3. Lösung nach Anspruch 2, wobei der Hilfsstoff eine polyhydroxylierte Verbindung ist, bevorzugt ein Zuckeralkohol, am stärksten bevorzugt Mannitol.

4. Lösung nach einem beliebigen der Ansprüche 1-3, wobei die Konzentration von Bendamustin in der Prälyophilisierungslösung 3-30 mg/ml, vorteilhafter 10-20 mg/ml beträgt, berechnet als Bendamustinhydrochlorid.

5. Lösung nach einem beliebigen der Ansprüche 1-4, wobei das Verhältnis zwischen Mannitol und Bendamustinhydrochlorid im Bereich von 1 : 1 bis 2 : 1 (Gew./Gew.), bevorzugt im Bereich von 1,5 : 1 bis 1,8 : 1 (Gew./Gew.) liegt.

6. Lösung nach einem beliebigen der Ansprüche 1-5, die eine sterile Lösung ist.

7. Verfahren zum Herstellen der Prälyophilisierungslösung gemäß einem beliebigen der Ansprüche 1-6, das Auflösen eines wasserlöslichen Salzes von Bendamustin und, gegebenenfalls, mindestens einem pharmazeutisch verträglichen Hilfsstoff in einem Lösungsmittelsystem, das Wasser im Gemisch mit Essigsäure umfasst, bei einer Temperatur, die nicht die Umgebungstemperatur übersteigt, und Filtern der Lösung umfasst.

8. Verfahren nach Anspruch 7, wobei das Auflösen in der Abwesenheit von Licht und/oder unter einer Inertgasatmosphäre durchgeführt wird.

9. Verfahren zum Herstellen einer pharmazeutischen Zusammensetzung, das Unterziehen der Prälyophilisierungslösung gemäß einem beliebigen der Ansprüche 1-6 einer Lyophilisierung umfasst.

10. Verfahren nach Anspruch 9, das Vorkühlen einer Lösung von Bendamustinhydrochlorid in Wasser-Essigsäure, Vorkühlen der Lösung auf eine Temperatur zwischen -50°C und -65°C, Annealing bei einer Temperatur zwischen -28°C bis -35°C, und Wiederabkühlen auf ei ne Temperatur zwischen -50°C und -65°C umfasst.

## Revendications

1. Une solution de pré-lyophilisation comprenant un sel hydrosoluble de bendamustine de formule I : 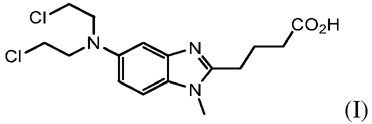 de préférence le chlorhydrate de bendamustine, dissous dans un système de solvants comprenant de l'eau en mélange avec 20 à 40% en volume d'acide acétique.

2. La solution selon la revendication 1, comprenant en outre au moins un excipient pharmaceutiquement acceptable.

3. La solution selon la revendication 2, dans laquelle l'excipient est un composé polyhydroxylé, de préférence un alcool de sucre, plus préférentiellement le mannitol.

4. La solution selon l'une quelconque des revendications 1 à 3, dans laquelle la concentration de la bendamustine dans la solution de pré-lyophilisation est de 3-30 mg/ml, plus avantageusement de 10-20 mg/ml, calculée en tant que chlorhydrate de bendamustine.

5. La solution selon l'une quelconque des revendications 1 à 4, dans laquelle le rapport mannitol/chlorhydrate de bendamustine se situe dans la plage allant de 1/1 à 2/1 (pds/pds), de préférence dans la gamme de 1,5/1 à 1,8/1 (pds/pds).

6. La solution selon l'une quelconque des revendications 1 à 5, qui est une solution stérile.

7. Un procédé de préparation de la solution de pré-lyophilisation selon l'une quelconque des revendications 1 à 6, comprenant la dissolution d'un sel hydrosoluble de bendamustine et, facultativement, au moins un excipient pharmaceutiquement acceptable, dans un système de solvant comprenant de l'eau en mélange avec de l'acide acétique à une température ne dépassant pas la température ambiante et la filtration de la solution.

8. Un procédé selon la revendication 7, dans lequel la dissolution est effectuée en l'absence de lumière et/ou sous une atmosphère d'un gaz inerte.

9. Un procédé de préparation d'une composition pharmaceutique, comprenant la soumission de la solution de pré-lyophilisation selon l'une quelconque des revendications 1 à 6 à la lyophilisation.

10. Le process selon la revendication 9, comprenant le pré-refroidissement d'une solution de chlorhydrate de bendamustine dans un mélange eau/acide acétique à une température comprise entre -50°C et -65°C, malléabilisation à une température comprise entre -28°C et -35°C, puis à nouveau refroidissement à une température comprise entre -50°C et -65°C.
